# EUROPEAN PATENT APPLICATION

(11) **EP 3 395 399 A1**
(43) Date of publication of application: **31.10.2018**
(21) Application number: 17193057.1
(22) Date of filing: 26.09.2017
(51) Int. Cl.: A61N 1/04

(54) **MUSCLE ELECTROSTIMULATION DEVICE**

(30) Priority: 27.04.2017 JP 2017088040
(71) Applicant: MTG Co., Ltd., Nagoya Aichi 453-0041 (JP)
(72) Inventor: MATSUSHITA, Tsuyoshi, Nagoya, Aichi 453-0041 (JP)
(74) Representative: Noble, Nicholas

(57) **Abstract**

A muscle electrostimulation device (10) includes: a main body (20) having a contact surface (10b) fitted around a portion fitted with the device (10) of a lower limb (6) or an upper limb. The contact surface (10b) extends in an X direction encircling the portion fitted with the device (10) and in a Y direction perpendicular to the X direction. The main body (20) is provided with a pair of electrodes (32) arranged at a distance in the Y direction. Each of the pair of electrodes (32) is shaped in a band in which an extent in the X direction is larger than an extent in the Y direction and is arranged to cause an electric current to flow in the portion fitted with the device (10) along a direction of extension of a muscle fiber in the portion fitted with the device (10).

## Description

The present invention relates to muscle electrostimulation devices.

A muscle electrostimulation device is known as one example of devices for training muscle. A muscle electrostimulation device causes an electric current to flow in the muscle fiber to stress or relax the muscle. This is expected to grow the muscle. For example, a muscle electrostimulation device described in patent document 1 is proposed. The muscle electrostimulation device described in patent document 1 is attached to, for example, the abdomen and stimulates the muscle there.
[patent document 1] JP2017-6644

We have gained the following knowledge regarding muscle electrostimulation devices. A cordless muscle electrostimulation device is desirable to secure the degree of freedom of the upper limb or the lower limb during training. One conceivable approach to address this is to build a primary battery or a secondary battery in the muscle electrostimulation device to drive the muscle electrostimulation device by using the battery. If the period of time elapsed before the battery capacity is consumed is short, it may result in short cycles of changing or charging the battery and poor usability.

In order to extend the cycle of changing or charging the battery, the capacity of the battery built in may be increased. However, the muscle electrostimulation device is worn on the body and so reduction in size and weight thereof is called for. If the capacity of the battery built in is increased, the size and weight of the battery will be increased, which may run contrary to the need for reduction in size and weight.

Based on the foregoing, we have recognized that there is room for improvement in muscle electrostimulation devices in terms of efficient electrostimulation of the muscle.

In this background, a general purpose of the present invention is to provide a muscle electrostimulation device capable of supplying electrostimulation to the muscle efficiently.

To address the issue, the muscle electrostimulation device according to an embodiment of the present invention comprises: a main body having a contact surface fitted around a portion fitted with the device of a lower limb or an upper limb. The contact surface extends in an X direction encircling the portion fitted with the device and in a Y direction perpendicular to the X direction, the main body is provided with a pair of electrodes arranged at a distance in the Y direction, and each of the pair of electrodes is shaped in a band in which an extent in the X direction is larger than an extent in the Y direction and is arranged to cause an electric current to flow in the portion fitted with the device along a direction of extension of a muscle fiber in the portion fitted with the device.

According to the embodiment, the band-shaped electrodes in which the extent in the X direction is larger than the extent in the Y direction allow an electric current to flow in the portion fitted with the device along the direction of extension of the muscle fiber.

Optional combinations of the aforementioned constituting elements, and replacements of constituting elements and implementations of the invention between methods, apparatuses, and systems may also be practiced as additional modes of the present invention.

Embodiments will now be described, by way of example only, with reference to the accompanying drawings which are meant to be exemplary, not limiting, and wherein like elements are numbered alike in several Figures, in which:
Fig. 1 is a schematic diagram showing the vicinity of a pair of electrodes attached to a portion fitted with the device;
Figs. 2A and 2B are schematic diagrams showing a training system employing the muscle electrostimulation device according to the first embodiment;
Fig. 3 is a block diagram showing functional features of the training system of Figs. 2A and 2B;
Fig. 4 is a front view of the muscle electrostimulation device of Figs. 2A and 2B;
Fig. 5 is a plan view of the muscle electrostimulation device of Figs. 2A and 2B;
Fig. 6 is a rear view of the muscle electrostimulation device of Figs. 2A and 2B;
Fig. 7 is a rear view showing an exemplary arrangement of leads of the muscle electrostimulation device of Figs. 2A and 2B;
Fig. 8 is a schematic diagram showing the muscle electrostimulation device of Figs. 2A and 2B fitted to the back of the upper leg portion;
Fig. 9 is a plan view showing an example of belt members of the muscle electrostimulation device of Figs. 2A and 2B;
Fig. 10 is a flowchart showing an example of the operation of the muscle electrostimulation of Figs. 2A and 2B;
Fig. 11 is a front view of the muscle electrostimulation device according to the second embodiment;
Fig. 12 is a block diagram showing features of the muscle electrostimulation device of Fig. 11;
Figs. 13A and 13B are side views schematically showing the vicinity of an attachment mechanism of the muscle electrostimulation device of Fig. 11;
Fig. 14 is a rear view showing a mount sheet pasted to the muscle electrostimulation device of Fig. 11; and
Figs. 15A and 15B are schematic diagrams illustrating a belt member according to the fourth variation.

The invention will now be described by reference to the preferred embodiments. This does not intend to limit the scope of the present invention, but to exemplify the invention.

We have gained the following knowledge regarding muscle electrostimulation devices attached to the upper limb or the lower limb for use, from the perspective of stimulating the muscle efficiently.

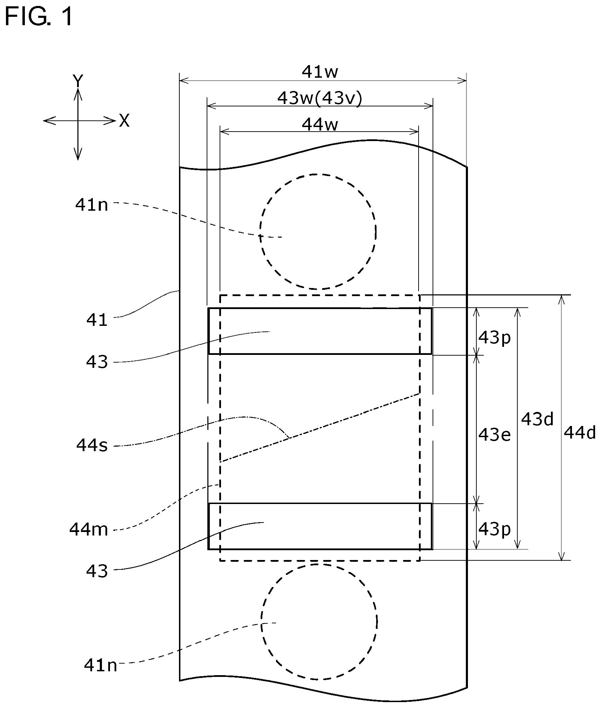

In many cases, the upper limb and the lower limb can be regarded as shaped in a cylinder with a diameter progressively smaller toward the end. Fig. 1 is a schematic diagram showing the vicinity of a pair of electrodes 43 attached to a cylindrical portion 41 fitted with the device. A main muscle 44m of the portion 41 fitted with the device has a muscle width 44w along the circumferential direction of the cylindrical shape (hereinafter, referred to as the X direction) and a muscle length 44d along the direction of extension of the cylindrical shape (hereinafter, referred to as the Y direction). The muscle fiber of the muscle 44m extends in the Y direction along the front and back of the bone near the center between two joints 41n. The muscle width 44w of the muscle 44m is considered to be slightly smaller than a width 41w of the semiperimeter of the portion 41 fitted with the device, and the muscle length 44d is considered to be slightly smaller than a distance between the two joints 41n. A nerve 44s controlling the muscle runs through the muscle 44m. In order to train the muscle 44m it is effective to electrically stimulate the nerve 44s distributed in a range defined by the muscle width 44w and the muscle length 44d. Based on the foregoing, the width of the placement area of the electrodes 43 in the X direction (hereinafter, referred to as an area width 43w) may be configured to be larger than the muscle width 44w, and, for example, configured in conformity to the width 41w of the semiperimeter of the upper limb or the lower limb.

A consideration is now be given of the length of the placement area of the electrodes 43 in the Y direction (hereinafter, referred to as an area length 43d). The area length 43d may be configured to be within the range of the muscle length 44d. However, if the electrodes 43 are too close to the joint 41n near the portion fitted with the device, the electrodes 43 may pressure the joint 41n strongly when the joint 41n is bent. For this reason, it is desired that the area length 43dbe configured to be smaller than the muscle length 44d to some degree. The pair of electrodes 43 may be formed to be accommodated in a rectangular range defined by the area width 43w and the area length 43d, which is determined by the conditions above.

According to our study, the length of the electrodes 43 in the Y direction (hereinafter, referred to as an electrode length 43p) is excessively large, stimulation felt by the body may be weak. Conceivably, this is because the electric current stimulating the nerve 44s governing the muscle 44m is not increased if the electrode length 43p is extended. It is also considered that the electric current flowing in the body may be increased and the life of the battery for which the battery is capable of carrying an electric current maybe shortened, if the electrode length 43p is extended. In essence, it is desired that the electrode length 43p be configured to be within a range capable of covering the nerve 44s governing the muscle.

From the perspective of causing an electric current to flow in the nerve 44s in an extensive range in the Y direction, it is advantageous to reduce the electrode length 43p and extend a distance 43e between the opposite edges of the pair of electrodes 43commensurately. Too short an electrode length would, however, result in unstable contact with the body. It is therefore desired to reduce the electrode length 43p the extent that the contact does not become unstable. Based on the foregoing, each of the pair of electrodes 43 may be shaped in a band in which the extent in the Y direction is larger than the extent in the X direction. In essence, the longitudinal direction of the pair of electrodes 43 may be arranged along the X direction to allow the pair of electrodes 43 to cause an electric current to flow in the portion 41 fitted with the device in the Y direction. By arranging the electrodes in this way, it is possible to cause an electric current to flow in the portion 41 fitted with the device along the direction of extension of the muscle fiber. Each of the pair of electrodes 43 may be substantially band-shaped. For example, the electrode may be of a shape such as a gourd shape including irregularities or curved portions at the circumference, a shape such as a tapered shape or a trapezoidal shape including non-parallel opposite sides, a shape such as an elliptical or oblong shape including an elliptical arc or a circular arc, etc.

A consideration will now be given of an angle of arrangement of the pair of rectangular shaped electrodes 43. From the perspective of extending a range in which it is possible to supply electrostimulation to the nerve 44s, it is desired that the longitudinal direction of the pair of electrodes 43 be arranged substantially parallel to the X direction, as shown in Fig. 1. However, the pair of electrodes 43 may be arranged at an angle to conform to the three-dimensional shape of the muscle 44m or the joint 41n. By way of example, the distance between the pair of electrodes 43 may be configured to be progressively smaller from one end along the Y direction toward the other end. In essence, the pair of electrodes 43 may be arranged in the shape of a letter V with its apical end missing, by approximating one end of one of the pair of electrodes 43 and one end of the other electrode. If the angle formed by the longitudinal directions of the pair of electrodes 43 is too large, one end of one of the pair of electrodes 43 approaches one end of the other electrode excessively, possibly resulting in an uneven electric current distribution and a narrow range in which the nerve 44s is stimulated. It is confirmed that practical performance is obtained in these perspectives if the angle formed by the longitudinal directions of the pair of electrodes 43 is in a range of 90° or less.

A further consideration is given of an electrode width 43v, which is the width of the electrodes in the X direction. In reality, the pair of electrodes are fixed on an insulative sheet and is wrapped around, for example, the semiperimeter of the portion 41 fitted with the device. A belt or the like may be joined to the sheet so as to fix the sheet on the portion 41 fitted with the device in a stable manner. For this purpose, it is desired that the electrode width 43v be reduced by an amount determined by a member for joining the belt. In essence, the electrode width 43v may be of a length derived by subtracting the width of the member for joining the belt from the width 41w of the semiperimeter of the portion 41 fitted with the device. The electrode width 43v may be configured to be identical to the area width 43w.

By configuring the shape of the pair of electrodes 43 based on the perspectives described above, it is possible to efficiently and effectively train a plurality of muscles 44m such as the quadriceps muscle of the thigh over a wide range. By using the electrodes configured as described above, it is possible to train the muscle on the front and the muscle on the back of the portion 41 fitted with the device.

The embodiments are devised based on the thinking described above and a description will now be given of the specific configuration thereof.

The present invention will be described based on preferred embodiments with reference to the drawings. In the embodiments and variations, the same or equivalent constituting elements and members shall be denoted by the same reference numerals, and duplicative explanations will be omitted appropriately. The dimension of members in the drawings shall be enlarged or reduced as appropriate to facilitate understanding. Those of the members that are not material to the description of the embodiments are omitted in the drawings.

Terms including ordinal numbers like first and second are used to describe a series of constituting elements, but those terms are used solely for the purpose of distinguishing one constituting element from another and shall not limit the constituting elements.

### [First embodiment]

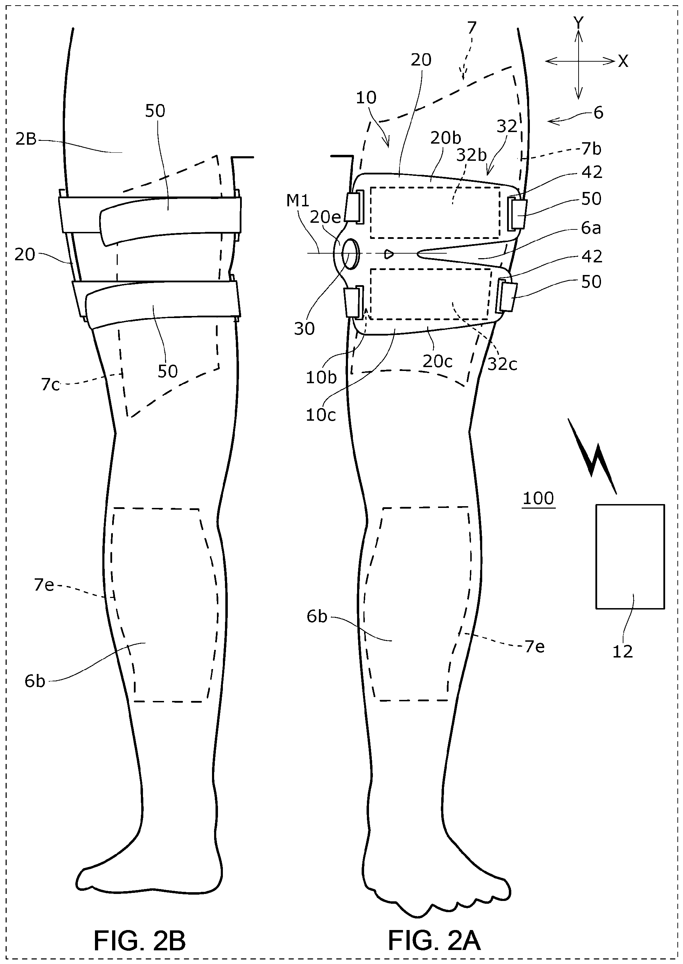

A description will be given of a muscle electrostimulation device 10 according to the first embodiment with reference to Figs. 2A-10. The muscle electrostimulation device 10 is a device to train the muscle of the upper limb such as the biceps muscle of arm and the triceps muscle of arm, and the muscle of the lower limb such as the quadriceps muscle, hamstring, and the triceps surae muscle. A description will now be given of an example of training the muscle of the lower limb.

Figs. 2A and 2B are schematic diagrams showing a training system 100 employing the muscle electrostimulation device 10 according to the first embodiment. Referring to Figs. 2A-2B, Fig. 2A shows a lower limb 6 viewed from front, and Fig. 2B shows the lower limb 6 viewed from back. In particular, the muscle electrostimulation device 10 can be used for a front muscle 7b of an upper leg portion 6a such as the quadriceps muscle, a back muscle 7c of the upper leg portion 6a such as the hamstring, and a muscle 7e of a lower leg portion 6b such as the triceps surae muscle. In the example of Figs. 2A-2B, the muscle electrostimulation device 10 is attached to the upper leg portion 6a of the user to supply electrostimulation to the muscle 7b. The muscle electrostimulation device 10 according to the first embodiment can similarly be used for the muscle 7e of the lower leg portion 6b.

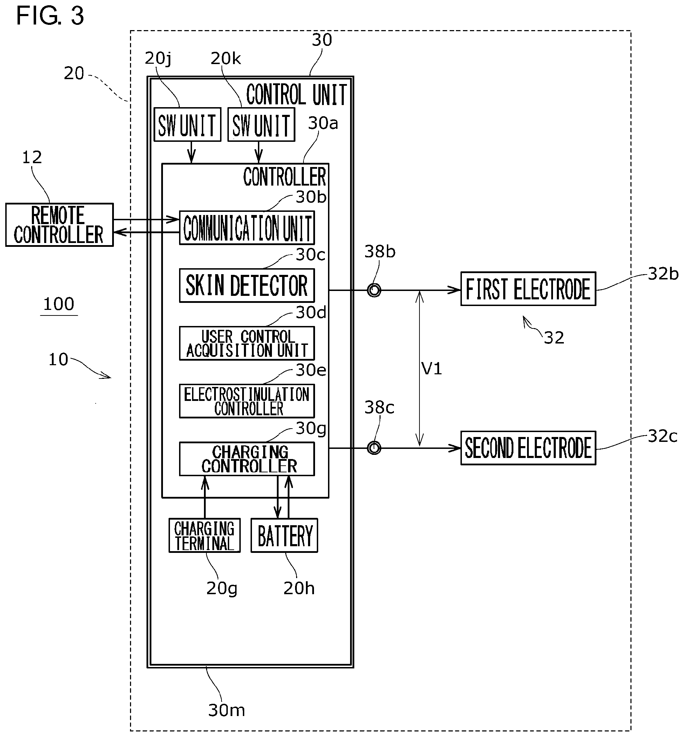

Fig. 3 is a block diagram showing functional features of the training system 100 employing the muscle electrostimulation device 10. The training system 100 includes the muscle electrostimulation device 10 and a remote controller 12.

The remote controller 12 is any of a variety of terminals manipulated by the user. For example, the remote controller 12 may be a PC, a tablet, or a smartphone. The remote controller 12 controls the operation of the muscle electrostimulation device 10 in accordance with user manipulation. The remote controller 12 receives the result of detection of the operating condition from the muscle electrostimulation device 10. By way of example, the muscle electrostimulation device 10 may transmit device information to the remote controller 12 by Bluetooth (registered trademark) or Wi-Fi (registered trademark) and receive user control information from the remote controller 12.

Further, the muscle electrostimulation device 10 detects an operating condition of the muscle electrostimulation device 10 and outputs a result of detection to the remote controller 12 by wireless or wired connection.

The remote controller 12 displays information on a touch panel in accordance with the operating condition obtained from the muscle electrostimulation device 10. The user can know the operating condition of the muscle electrostimulation device 10 by viewing the display on the touch panel of the remote controller 12. The remote controller 12 is adapted to detect user control on the touch panel. The user can provide a touch operation by taping the touch panel once lightly with a finger or swiping in a certain direction on the touch panel with a finger pressed against the touch panel. The remote controller 12 is adapted to generate user control information based on the user's touch operation and transmit the information to the muscle electrostimulation device 10.

As shown in Figs. 2A-2B, the muscle electrostimulation device 10 according to the first embodiment is fitted around the upper leg portion 6a of the lower limb 6 for use. The muscle electrostimulation device 10 has a contact surface 10b fitted around the upper leg portion 6a when the muscle electrostimulation device 10 is worn. The contact surface 10b extends in the X direction and in the Y direction perpendicular to the X direction when the contact surface 10b is expanded along the flat surface. The X direction and the Y direction merely define the orientation of the surface of the contact surface 10b. The contact surface 10b may include a portion extending in a direction inclined to the Y direction. The direction perpendicular to the X direction and the Y direction is defined as the Z direction (not shown in Figs. 2A-2B). The Z direction is perpendicular to the contact surface 10b.

In the example of Figs. 2A-2B, the muscle electrostimulation device 10 is worn such that the Y direction of the contact surface 10b is aligned with the direction of extension (vertical direction) of the lower limb 6 and the X direction of the contact surface 10b encircles the upper leg portion 6a. Hereinafter, the Y direction may be denoted as the upward direction or the downward direction, and the Z direction may be denoted as the thickness direction. The notation of directions above shall not limit the orientation of the muscle electrostimulation device 10 in use, and the muscle electrostimulation device 10 may be used in any orientation.

The muscle electrostimulation device 10 includes a main body 20 and two belt members 50 spaced apart in the Y direction. The main body 20 is guided around the front or back of the upper leg portion 6a. The belt members 50 are guided from one end of the main body 20, are guided around the back or front of the upper leg portion 6a in the circumferential direction, and are joined to the other end of the main body 20. The belt members 50 will be described later.

The main body 20 is provided with a control unit 30, belt joints 42, and a pair of electrodes 32 arranged at a distance in the Y direction. The control unit 30 is an electronic unit for supplying a voltage for muscle stimulation to the pair of electrodes 32. The belt joints 42 are members for joining the two belt members 50 to the main body 20. The pair of electrodes 32 are sheet electrodes that supply a voltage for muscle stimulation to the body. The pair of electrodes 32 include a first electrode 32b and a second electrode 32c provided at a distance in the Y direction. In this example, the first electrode 32b and the second electrode 32c are provided such that the longitudinal directions thereof are parallel to the X direction.

Two band-shaped members 20b, 20c extending in the X direction are arranged in the Y direction so that the main body 20 is substantially shaped in a rectangle as a whole. The main body 20 may be substantially shaped in a square. The two band-shaped members 20b, 20c may be joined in some portions and separated in the other portions. A pair of belt joints 42 may be provided at the respective ends of each of the two band-shaped members 20b, 20c in the X direction.

The control unit 30 may be provided at the center of the main body 20 in the Y direction and proximate to one end of the main body 20 in the X direction. The center of the main body 20 may be located on a straight line M1 bisecting the main body 20 in the Y direction and extending in the X direction. The control unit 30 may be provided in an overhanging portion 20e overhanging from the end of the main body 20 in the X direction. The main body 20 may be formed in symmetry with respect to the control unit 30, by including the two band-shaped members 20b, 20c. In particular, the main body 20 may be formed in line symmetry with respect to the straight line M1. The pair of electrodes 32 may be arranged in symmetry with respect to the control unit 30. In particular, the pair of electrodes 32 may be arranged in line symmetry with respect to the straight line M1. By having a symmetrical shape as described above, the muscle electrostimulation device 10 as used for the upper limb or the lower limb can be suitably used for respective portions merely by reversing the orientation of the device.

In this example, the main body 20 is attached to the upper leg portion 6a. The pair of electrodes 32 are configured to cause an electric current for muscle stimulation to flow in the muscle 7b of the upper leg portion 6a along the Y direction. In essence, the pair of electrodes 32 are arranged to cause a circumferential electric current to flow in the muscle 7b of the upper leg portion 6a along the direction of extension of the muscle fiber.

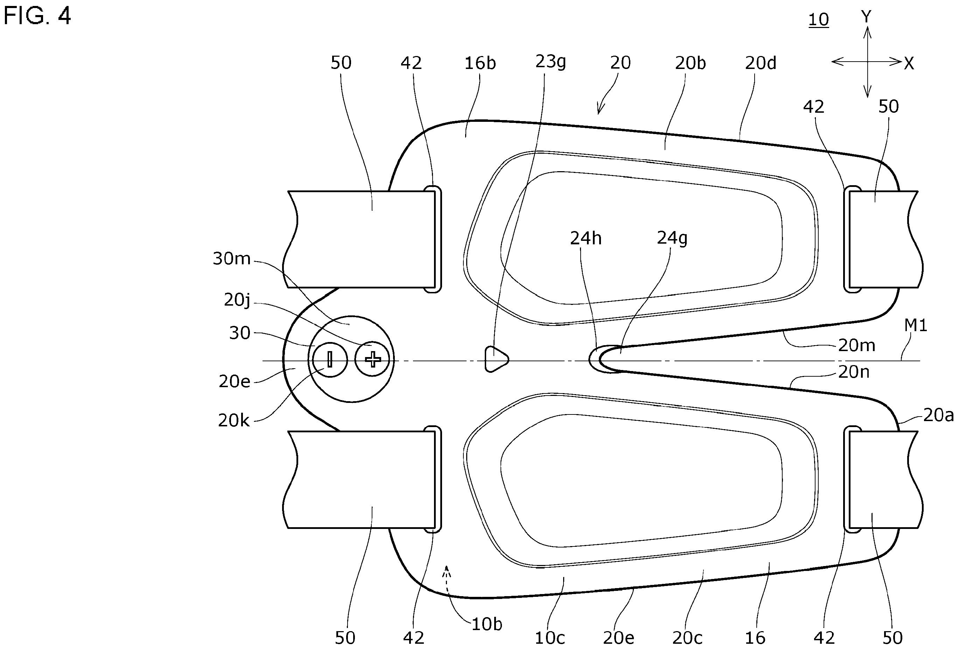

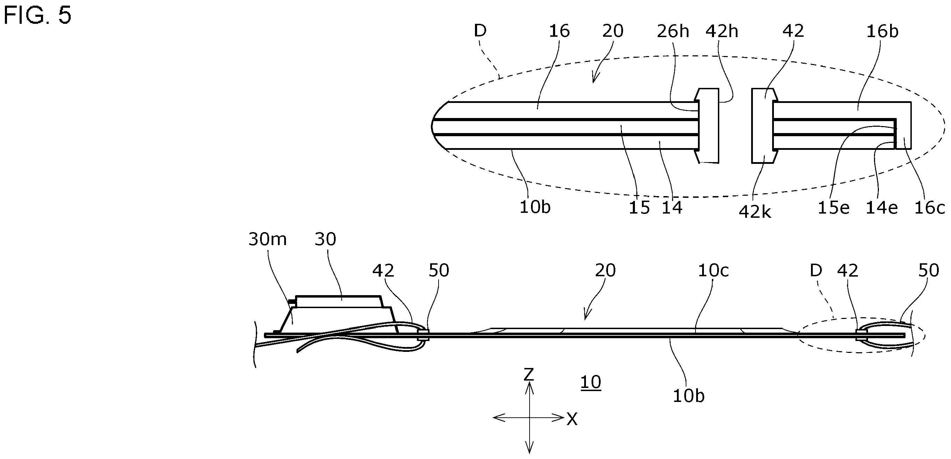

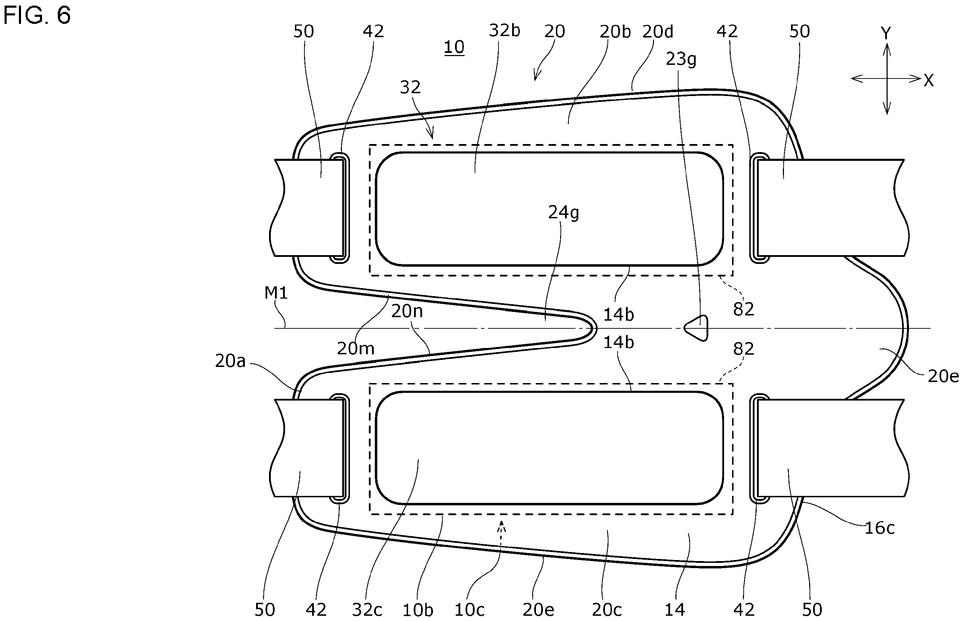

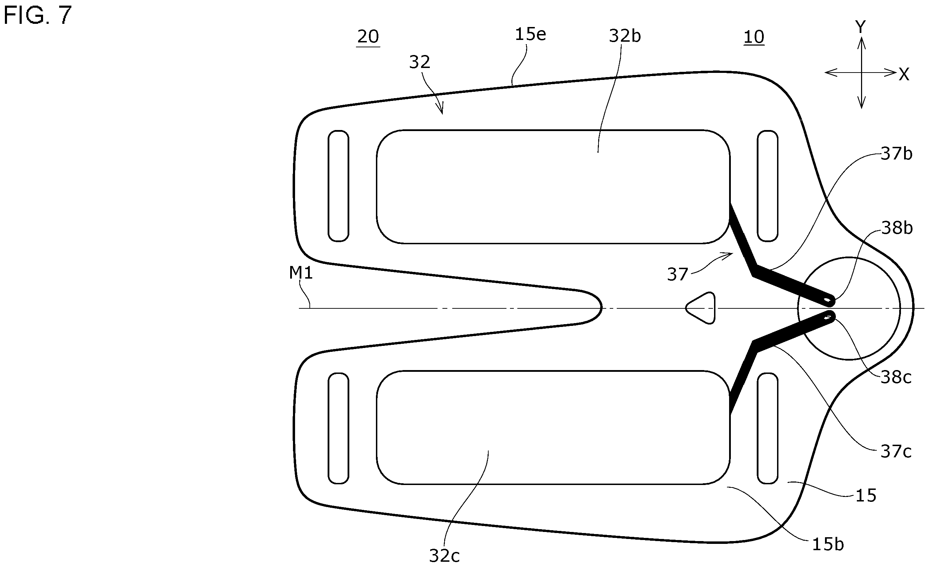

Fig. 4 is a front view of the muscle electrostimulation device 10. Fig. 5 is a plan view of the muscle electrostimulation device 10. Fig. 6 is a rear view of the muscle electrostimulation device 10. Fig. 7 is a rear view showing an exemplary arrangement of leads 37 of the muscle electrostimulation device 10. Figs. 4-7 are front views of the contact surface 10b of the respective units by expanding the muscle electrostimulation device 10. As shown in Figs. 4-7, the X direction, which corresponds to the circumferential direction of the lower limb 6, and the Y direction, which corresponds to the vertical direction of the lower limb 6, are indicated by straight lines of arrows X and Y in an expanded state.

### (Main body)

The main body 20 may include a layered structure. By way of example, the main body 20 includes a first base member 14, a second base member 15, and a cover member 16. The first base member 14 is a member fitted to the lower limb 6 and exhibits a flat sheet shape when expanded. A contact surface 10b in contact with the upper leg portion 6a is provided on the back surface of the first base member 14. The first base member 14 has an opening 14b that exposes the pair of electrodes 32 on the contact surface 10b. The first base member 14 may be configured by combining a plurality of members. The durability of the leads 37 of the muscle electrostimulation device 10 of this example is ensured to be higher than when the first base member 14 is not provided.

The second base member 15 is a member supporting the pair of electrodes 32 and the leads 37 and exhibits a flat sheet shape when expanded. The second base member 15 may be configured by combining a plurality of members. The second base member 15 of this example is configured such that the portions corresponding to main body 20 are integrated. The rear surface of the second base member 15 is provided with the pair of electrodes 32. For example, the pair of electrodes 32 can be formed by printing an electroconductive material such as silver paste. The pair of electrodes 32 may be formed by etching. The second base member 15 is provided with the leads 37 electrically connected to the pair of electrodes 32. For example, the leads 37 can be formed by printing an electroconductive material such as silver paste. The leads 37 may be formed by etching. The leads 37 may be referred to as a wiring pattern.

The cover member 16 is a member built on the side of the second base member 15 opposite to the first base member 14. The cover member 16 includes a sheet portion 16b exhibiting a flat sheet shape when expanded, and an edge cover portion 16c extending from the outer edge of the sheet portion 16b toward the secondbase member 15 in the thickness direction (Z direction) . An outer surface portion 10c of the sheet portion 16b of the cover member 16 is provided with the control unit 30. The cover member 16 may be configured by combining a plurality of members. The cover member 16 of this example is configured to cover the main body 20 for integration.

By forming the first base member 14 and the second base member 15 to be thin, outer edges 14e and 15e thereof will also be thin. When the muscle electrostimulation device 10 is worn on the lower limb 6, the outer edges 14e, 15e may be in contact with the lower limb 6, giving the user an uncomfortable tactile sensation. To address this, the cover member 16 of this example has the edge cover portion 16c that covers the outer edges 14e, 15e of the first base member 14 and the second base member 15. According to this configuration, the edge cover portion 16c covers the outer edges 14e, 15e so that the area of contact between the main body 20 and the lower limb 6 is increased and the tactile sensation experienced when the device is worn is improved. The edge cover portion 16c of this example is shaped in a band encircling the outer edges 14e, 15e. The rigidity of the cover member 16 of this example is ensured to be higher than when the edge cover portion 16c is not provided. Additionally, when the muscle electrostimulation device 10 is worn on the body, the outer edges 14e, 15e maybite into the skin. Byproviding the edge cover portion 16c, the stimulus and pain on the skin can be mitigated.

The embodiment is non-limiting as to the material of the first base member 14 and the second base member 15. Any insulative material that is flexible enough to be wrapped around the lower limb 6 may serve the purpose. The first base member 14 and the second base member 15 of this example are made of an elastomer such as polyethylene terephthalate-based resin. The embodiment is non-limiting as to the material of the cover member 16. Any insulative material that is flexible enough to be wrapped around the lower limb 6 may serve the purpose. By way of example, the cover member 16 may be made of a resin material harder than the first base member 14 and the second base member 15. The cover member 16 of this example is made of an elastomer such as silicon resin. In this case, the cover member 16 protects the first base member 14 and the second base member 15, inhibits damage from handling, and is capable of imparting the main body 20 with suitable rigidity. By using the muscle electrostimulation device 10, the body temperature is transmitted to the silicon resin and stored therein. Therefore, the user can feel the warmth while using the device, and it is expected that the blood flow is improved due to the hyperthermic effect.

For example, the main body 20 is fitted at a position of the lower limb 6 aligned with the muscle 7b of the upper leg portion 6a. The main body 20 extends in the X direction and in the Y direction and is formed to be thin in the Z direction. The main body 20 is provided with the pair of electrodes 32 arranged at a distance in the Y direction. As described above, the pair of electrodes 32 are configured to cause an electric current to flow in the muscle 7b of the upper leg portion 6a in a direction perpendicular to the circumferential direction. The main body 20 has a shape substantially considered as a rectangle in which the longitudinal direction is aligned with the X direction in a front view. Hereinafter, the shape that can substantially be considered as a rectangle includes a shape provided with a pair of opposite sides having a length of 30% or larger of the entire length.

The space between the muscle electrostimulation device 10 and the upper leg portion 6a may become humid due to the sweat during the training. To address this, the main body 20 of this example is provided with one or a plurality of air vent holes 23g formed at positions distanced from the pair of electrodes 32. As shown in Fig. 4, the air vent holes 23g are provided at positions distanced from the electrodes and the leads. In this case, the humidity between the muscle electrostimulation device 10 and the upper leg portion 6a is mitigated as compared with a configuration in which air vent holes are not provided. An additional benefit of providing the air vent holes 23g is that the flexibility of the main body 20 is improved. Bymodifying the number and arrangement of air vent holes 23g, the flexibility of the main body 20 can be adjusted.

In many cases, the circumferential surface of the upper leg portion 6a has the shape of a conical surface having a radius of curvature progressively larger toward the root. For this reason, wrinkles are formed in some portions of the main body 20 as it is fitted to the upper leg portion 6a, if the main body 20 is not provided with a bifurcation portion. When wrinkles are formed, the pair of electrodes 32 may be inclined to the circumferential surface of the upper leg portion 6a, portions of the electrodes 32 may be elevated, and the contact may become unstable. For this reason, a portion of the main body 20 in the X direction may branch in the Y direction into a plurality of portions so as to conform to different radiuses of curvature. By way of example, the main body 20 has a bifurcated shape in which a portion in the X direction is split in the Y direction to create a gap 24g. In essence, the main body 20 includes the two band-shaped members 20b, 20c spaced apart by the gap 24g. An edge 20m of the band-shaped member 20b is adjacent to the gap 24g. An edge 20n of the band-shaped member 20c is adjacent to the gap 24g. In essence, the gap 24g is an area sandwiched by the edges 20m, 20n in the Y direction. The band-shaped members 20b, 20c are joined in a part but separate in the other part. Each of the band-shaped members 20b, 20c of this example is shaped in a band in which the dimension in the X direction is larger than the dimension in the Y direction. The gap 24g is formed to face inward in the X direction from an end 20a of the main body 20 in the X direction.

The main body 20 of this example is formed in line symmetry with respect to a straight line extending in the X direction through the center of the gap 24g in the Y direction. In this example, the pair of electrodes 32 are provided in the respective areas spaced apart by the gap 24g of the main body 20. The band-shaped members 20b, 20c spaced apart by the gap 24g of the main body 20 are independently wrapped around the circumference of the upper leg portion 6a so that wrinkles are unlikely to be formed and the contact with the body can be stabilized. The bifurcated shape allows the band-shaped members 20b, 20c to be curved independently and reduces wrinkles, in the presence of a change in thickness such as that of the upper leg portion 6a. The dimension of the gap 24g in the Y direction, which is a depth of the cut of the bifurcated shape, may be large if the device is to be fit to the upper leg portion of the lower limb and may be small if the device is to be fit to the upper limb.

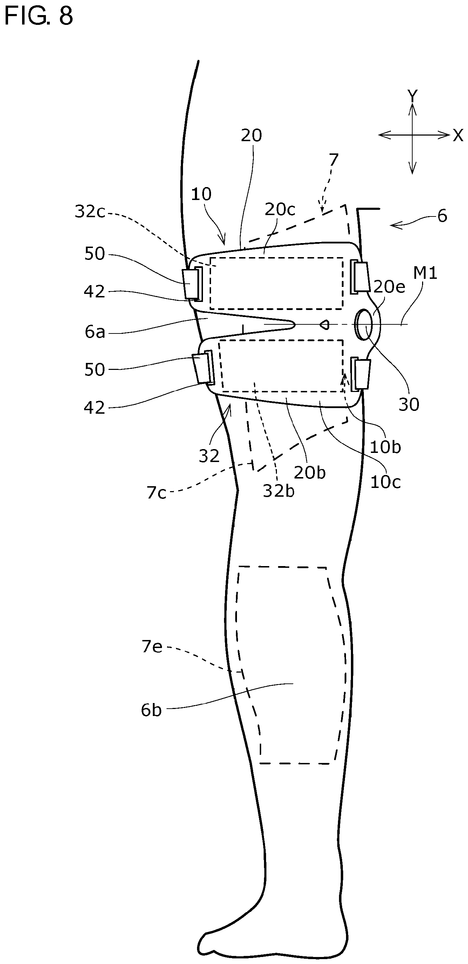

If the control unit 30 is outwardly arranged on the body, it is highly likely that the control unit 30 is damaged by colliding with, for example, a leg of a desk as the user moves. To address this, the muscle electrostimulation device 10 of this example is configured such that the control unit 30 can be inwardly arranged on the body whether the muscle electrostimulation device 10 is fitted to the front or back of the upper leg portion 6a. More specifically, the main body 20 is formed to be symmetrical with respect to the control unit 30. Fig. 8 is a schematic diagram showing the muscle electrostimulation device 10 fitted to the back of the upper leg portion 6a. The muscle electrostimulation device 10 in the state shown in Fig. 8 can train the muscle 7c on the back of the upper leg portion 6a. In this example, the main body 20 is fitted upside down with respect to the case of Fig. 2. By fitting the main body 20 in this way, the control unit 30 is inwardly arranged on the body so that the likelihood of the control unit 30 colliding with, for example, a leg of a desk and being damaged accordingly is reduced.

Reference is made back to Figs. 2-7. In the presence of the bifurcated portion split by the gap 24g, the stress may be concentrated in the portion of the main body 20 adjacent to the bottom of the gap 24g, which may result in a trouble such as a crack in that portion. To address this, the portion of the main body 20 of this example adjacent to the bottom of the gap 24g is provided with a thick portion 24h formed to have a larger thickness than elsewhere. In particular, the thick portion 24h may be provided around the portion of the main body 20 adjacent to the bottom of the gap 24g. The shape of the thick portion 24h may be defined by a simulation in accordance with the desired durability.

### (Leads)

As shown in Fig. 7, the leads 37 of this example are provided on a rear surface 15b of the second base member 15. The leads 37 include a wiring 37b and a wiring 37c. The wiring 37b electrically connects a terminal 38b and the first electrode 32b. The wiring 37c electrically connects a terminal 38c and the second electrode 32c.

### (Pair of electrodes)

As described above, the pair of electrodes 32 include the first electrode 32b and the second electrode 32c arranged at a distance in the Y direction. The longitudinal direction of each of the pair of electrodes 32 is arranged parallel to the X direction. Each of the pair of electrodes 32 is shaped in a band in which the extent in the X direction is larger than the extent in the Y direction. Each of the pair of electrodes 43 may be substantially band-shaped. For example, the electrode may be of a shape such as a gourd shape including irregularities or curved portions at the circumference, a shape such as a tapered shape or a trapezoidal shape including non-parallel opposite sides, a shape such as an elliptical or oblong shape including an elliptical arc or a circular arc, etc. In this example, each of the electrodes 32 is substantially rectangular-shaped. Each of the pair of electrodes 32 is arranged to extend in the X direction, which is a circumferential direction of the upper leg portion 6a, and causes an electric current in the Y direction to flow in the muscle 7b of the upper leg portion 6a. The pair of electrodes 32 are provided on the second base member 15 and are exposed on the contact surface 10b from the opening 14b provided in the first base member 14. The first electrode 32b is electrically connected to the terminal 38b by the wiring 37b, and the second electrode 32c is electrically connected to the terminal 38c by the wiring 37c.

When the muscle electrostimulation device 10 is fitted to the body for use, a gel pad 82 (see Fig. 6) is placed between the electrodes and the body. The gel pad 82 is sticky. The gel pad 82 plays the role of securing conductivity between the body and each electrode with its stickiness. The gel pad 82 is pasted to each electrode of the muscle electrostimulation device 10. The muscle electrostimulation device 10 is fitted to the body with the gel pad 82 pasted. The muscle electrostimulation device 10 is supported on the body by the stickiness of the gel pad 82. In this example, the gel pad 82 is pasted one each to the pair of electrodes 32 for use as shown in Fig. 6.

### (Control unit)

A description will now be given of the control unit 30. The blocks of the control unit 30 shown in Fig. 3 and those of the control unit 70 described later can be implemented in hardware such as devices or mechanical components exemplified by a Central Processing Unit (CPU) of a computer, and in software such as a computer program etc. Fig. 3 depicts functional blocks implemented by the cooperation of these elements. Therefore, it will be understood by those skilled in the art reading this specification that the functional blocks may be implemented in a variety of manners by a combination of hardware and software.

The control unit 30 includes a controller 30a, switch units 20j and 20k, a charging terminal 20g, and a battery 20h, which are housed in a housing 30m. The housing 30m is provided at the center of the main body 20 and is configured as an outer shell of the control unit 30. The housing 30m can be made of various resin materials. The switch units 20j, 2k are provided on the front of the control unit 30. The switch units may be denoted as SW units. The switch units 20j, 20k detect that the user holds down the switch and output a detection result to the controller 30a. The switch units 20j, 20k may include an electrical contact that is closed while the switch is not being held down and is opened when the switch is held down. The surface of the switch unit 20j of this example is labeled by a plus sign, and the surface of the switch unit 20k of this example is labeled by a minus sign.

The battery 20h is electrically connected to the controller 30a and supplies electric power to the controller 30a. The battery 20h may be a primary battery. In this example, a rechargeable lithium ion battery is employed. The battery 20h may be replaceable, but a built-in battery is employed in this example. The charging terminal 20g receives electric power to charge the battery 20h and outputs the power to the controller 30a. Various connectors can be used for the charging terminal 20g. In this example, a USB (registered trademark) connector is employed. For example, the charging terminal 20g is provided on the bottom surface of the control unit 30. The battery 20h is charged by the electric power received by the charging terminal 20g. The battery 20h may be configured to be charged by a charging system of non-contact type such as a wireless charging system.

The controller 30a includes a communication unit 30b, a skin detector 30c, a user control acquisition unit 30d, an electrostimulation controller 30e, and a charging controller 30g. The communication unit 30b is adapted to transmit an operating condition of the muscle electrostimulation device 10 to the remote controller 12. The remote controller 12 displays the operating condition acquired from the communication unit 30b. The communication unit 30b receives user control information based on a touch operation input in the remote controller 12. The controller 30a controls the operation of the muscle electrostimulation device 10 in accordance with the user control information acquired via the communication unit 30b.

The user control acquisition unit 30d acquires a detection result from the switch units 20j, 20k. The user control acquisition unit 30d detects the voltage commensurate with the resistance of the switch unit. If the detected voltage is equal to higher than a threshold value, the user control acquisition unit 30d determines that the switch unit is held down. If the detected voltage is less than the threshold value, the user control acquisition unit 30d determines that the switch unit is not held down. The controller 30a determines a method of controlling the muscle electrostimulation device 10 in accordance with a combination of statuses of the switch units indicating whether the switch is held down and with a duration of the switch unit being held down.

User control through the switch units 20j, 20k may be prohibited while the remote controller 12 is being manipulated. The muscle electrostimulation device 10 of this example is configured such that user control through the switch units 20j, 20k is enabled even when the remote controller 12 is being manipulated. In this case, the user can manipulate the muscle electrostimulation device 10 with the switch units 20j, 20k even when the user is away from the remote controller 12 and cannot use the remote controller 12 for manipulation.

The skin detector 30c detects whether the electrodes are in contact with the skin. The skin detector 30c detects the resistance between the pair of electrodes 32. The skin detector 30c determines that the electrodes are in contact with the skin when the detected resistance is less than a threshold value and determines that the electrodes are not in contact with the skin when the detected resistance value is equal to or higher than the threshold value.

When the skin detector 30c detects that the electrodes are in contact with the skin, the electrostimulation controller 30e supplies electric power commensurate with a preset output voltage to the pair of electrodes 32 for a predetermined duration of operation (e.g., 20 minutes) and at a predetermined interval. In essence, the upper leg portion 6a of the user is electrically stimulated for the duration of operation. The output voltage can be altered by manipulating the remote controller 12 or the switch units 20j, 20k. The output voltage in the electrostimulation controller 30e of this example rises each time the switch unit 20j is held down and drops each time the switch unit 20k is held down.

The charging controller 30g controls the electric power for charging received in the charging terminal 20g and supplies the electric power to the battery 20h. The charging controller 30g controls the magnitude of the electric current supplied to the battery 20h in accordance with the charging rate of the battery 20h. For example, if the charging rate is low, the charging controller 30g increases the electric current supplied to the battery 20h. If the charging rate is high, the charging controller 30g decreases the electric current supplied to the battery 20h. If a margin of increase in the charging rate is small in relation to the amount of charge stored, it is determined that battery has failed and the battery is not charged further.

### (Belt joints)

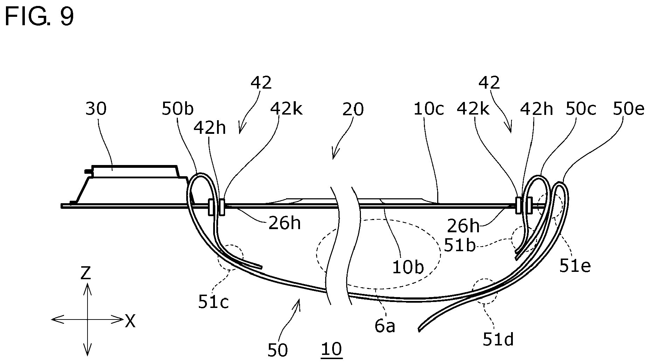

The main body 20 is provided with a pair of belt joints 42 for joining the belt members 50 to the two band-shaped members 20b, 20c respectively. Fig. 9 is a plan view showing an example of the two belt members 50 joined to the muscle electrostimulation device 10. For ease of understanding, Fig. 9 shows the main body 20 extended in the X direction and shows the upper leg portion 6a in a scale smaller than the actual scale. One end 50b of a belt of the belt members 50 is joined to one of the pair of belt joints 42, and the main body 20 is wrapped around the upper leg portion 6a, which is the portion fitted with the device. In this state, the other end of the belt of the belt members 50 is joined to the other of the pair of belt joints 42. More specifically, the ends of the belt of the belt members 50 guided around a portion of the upper leg portion 6a is fitted to the pair of belt joints 42.

It is desired that the muscle electrostimulation device 10 be easily attached to the body. To address this in this example, when one end 50b of a belt of the belt members 50 is joined to one of the pair of belt joints 42 and the main body 20 is wrapped around the portion fitted with the device, the other of the pair of belt joints 42 is located in a range in which the other end of the belt of the belt members 50 can be guided through the other of the pair of belt joints 42. More specifically, the pair of belt joints 42 are provided in each of the two band-shaped members 20b, 20c and spaced apart in the X direction. When the main body 20 is wrapped around in the X direction, the pair of belt joints 42 are aligned with the X direction. For this reason, the other end of the belt of the belt members 50 joined to one of the belt joints 42 is automatically located at the other of the pair of belt joints 42 so that the belt can be easily joined to the belt joints 42.

The belt joints 42 include a pair of rectangular tube members 42k. Each of the rectangular tube members 42k is a rectangular tube member encircling a hole 42a through which the belt of the belt members 50 is guided. Each of the rectangular tube members 42k substantially presents a shape of a rectangle extending longitudinally in the Y direction in a front view. A substantially rectangular hole 42h elongated in the Y direction is provided at the center. The rectangular tube members 42k may be made of a resin material by way of an example. The pair of rectangular tube members 42k are provided in each of the two band-shapedmembers 20b, 20c and spaced apart in the X direction. By using the rectangular tube members 42k, the force from the belt members 50 is received by the rectangular tube members 42k. Therefore, the load exerted on the cover member 16 is distributed and occurrence of cleavage or deformation in appearance of the belt members 50 is inhibited as contrasted with a case where the rectangular tube members 42k are not provided. Further, displacement in the layered structure of the first base member 14, the second base member 15, and the cover member 16 can be inhibited.

### (Belt members)

Variously configured members may be employed for the belt members. For example, the belt members may be configured as a cord, a band, etc. The belt members of this example are band-shaped members made of cloth and having a hook-and-loop fastener on both surfaces.

In this example, one of the pair of rectangular tube members 42k is fitted with a fastener member 50c. The belt of the belt members 50 bridges the other of the pair of rectangular tube members 42k and the fastener member 50c. In this example, the fastener member 50c is a band-shaped member made of cloth and having a hook-and-loop fastener. The fastener member 50c is guided through the hole 42h of one of the rectangular tube members 42k and is then folded back. The ends of the fastener member 50c are joined together by a hook-and-loop fastener 51b. The fastener member 50c may be formed to be shorter than the belt of the belt members 50.

One end of the belt of the belt members 50 is guided through the hole 42h of the other of the rectangular tube members 42k and is folded back. The end that is folded back is joined to an intermediate portion of the belt of the belt members 50 by a hook-and-loop fastener 51c. The other end of the belt of the belt members 50 is folded back for adjustment of the length (hereinafter, referred to as a folded-back portion 50e). The folded-back portion 50e, which is the other end of the belt of the belt members 50, is joined to another intermediate portion of the belt of the belt members 50 by a hook-and-loop fastener 51d.

The folded-back portion 50e is adapted to be joined to the fastener member 50c by a hook-and-loop fastener 51e. The muscle electrostimulation device 10 is fitted around the upper leg portion 6a of the user while the folded-back portion 50e is detached from the fastener member 50c. The muscle electrostimulation device 10 is fitted to the upper leg portion 6a by joining the folded-back portion 50e to the fastener member 50c while the muscle electrostimulation device 10 is fitted around the circumference of the upper leg portion 6a. By altering the position where the folded-back portion 50e is folded back, the circumferential length can be easily adjusted.

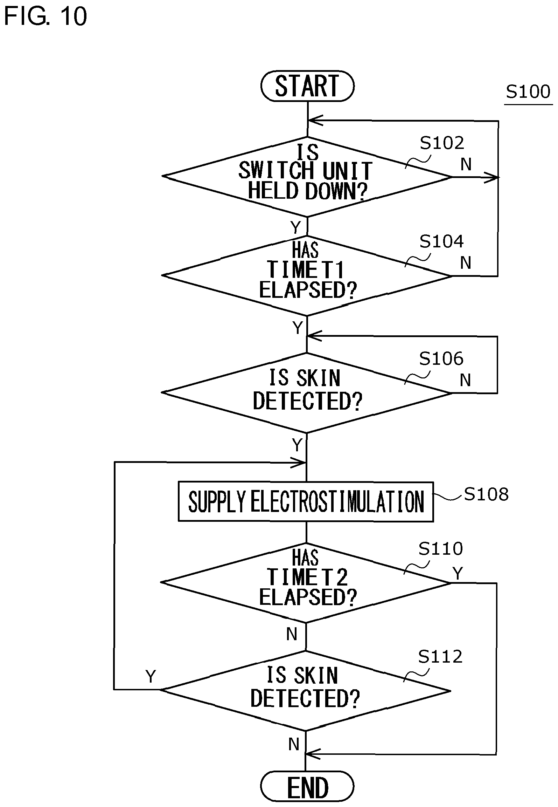

A description will be given of the operation of the muscle electrostimulation device 10 configured as described above. Fig. 10 is a flowchart showing an example of the operation of the muscle electrostimulation device 10. Fig. 10 shows a process S100 performed since power is turned on while the user is wearing the muscle electrostimulation device 10 on the upper leg portion 6a to start electrostimulation to the upper leg portion 6a until the electrostimulation is terminated.

When the process is started, the controller 30a determines whether the switch unit 20j is held down (step S102). If the switch unit 20j is not held down (N in step S102), the controller 30a returns control to step S102 and repeats step S102. If the switch unit 20j is being held down (Y in S102), the controller 30a determines whether a predetermined period of time T1 has elapsed (step S104).

If the period of time T1 has not elapsed (N in step S104), the controller 30a returns control to step S102 and repeats steps S102-S104. If the period of time has elapsed (Y in step S104), the controller 30a shifts to step S106. The period of time T1 in step S104 can be defined in accordance with a desired specification. The period of time T1 is defined to be 2 seconds in this example. In essence, the user can turn on the muscle electrostimulation device 10 by holding down the switch unit 20j for 2 seconds.

In step S106, the controller 30a determines whether the electrodes are in contact with the skin (step S106). If the electrodes are not in contact with the skin (N in S106), the controller 30a returns the process to step S106 after waiting a predetermined period of time.

If the electrodes are in contact with the skin (Y in S106), the controller 30a starts supplying electrostimulation to the upper leg portion 6a (step S108). In this step, the electrostimulation controller 30e supplies electric power that varies at a predetermined interval to the pair of electrodes 32.

The controller 30a having performed step S108 determines whether a predetermined period of time T2 has elapsed (step S110). The period of time T2 in step S110 can be defined in accordance with a desired training time. The period of time T2 is defined to be 20 minutes in this example. In essence, the user can continue training until 20 minutes elapses. If the period of time T2 has not elapsed (N in S110), the controller 30a determines whether the electrodes are in contact with the skin (step S112).

If the electrodes are in contact with the skin (Y in S112), the controller 30a returns the process to step S108 and continues to supply electrostimulation. If the electrodes are not in contact with the skin (N in S112), the controller 30a discontinues electrostimulation and terminates the process S100. In essence, the controller 30a discontinues electrostimulation once the electrodes are detached from the body 2, even if the period of time T2 has not elapsed. If the period of time T2 has elapsed (Y in S110), the controller 30a discontinues electrostimulation and terminates the process S100.

The process S100 is by way of example only and other steps may be added, or steps may be deleted or modified, or the sequence of steps may be switched.

A description will now be given of the advantage and benefit of the muscle electrostimulation device 10 according to the first embodiment configured as described above.

The muscle electrostimulation device 10 according to the first embodiment includes a main body 20 having a contact surface 10b fitted around the portion of the lower limb or the upper limb fitted with the device. The contact surface 10b extends in the X direction encircling the portion fitted with the device and in the Y direction perpendicular to the X direction. The main body 20 is provided with a pair of electrodes 32 arranged at a distance in the Y direction. Each of the pair of electrodes 32 is shaped in a band in which the extent in the X direction is larger than the extent in the Y direction and is arranged to cause an electric current to flow in the portion fitted with the device along the direction of extension of the muscle fiber in the portion fitted with the device. According to this configuration, the extent of the electrodes in the X direction is relatively large so that the muscle can be stimulated in an extensive range in the X direction. Further, since the extent of the electrodes in the Y direction is relatively small, the electric current flowing in the body can be inhibited and the life of the battery for which the battery is capable of carrying an electric current can be extended as compared with the case where the extent is large. Causing an electric current to flow in the Y direction in the portion attached with with the device allows the electric current to flow in the direction of extension of the muscle. Thus, the nerve that governs the muscle can be efficiently simulated and the muscle can be trained efficiently. This allows the muscle to be electrically stimulatedefficiently.

In the muscle electrostimulation device 10 according to the first embodiment, the longitudinal directions of the pair of electrodes 32 are arranged substantially parallel to each other. According to this configuration, a wider range of muscle in the portion fitted with the device can be stimulated.

In the muscle electrostimulation device 10 according to the first embodiment, the main body 20 is provided with air vent holes 23g formed at positions distanced from the pair of electrodes 32. According to this configuration, the humidity between the muscle electrostimulation device 10 and the upper leg portion 6a is mitigated as compared with a configuration in which air vent holes are not provided.

In the muscle electrostimulation device 10 according to the first embodiment, the main body 20 includes the first base member 14, the second base member 15, and the cover member 16. The first base member 14 has the opening 14b that exposes the pair of electrodes 32. The second base member 15 is provided with the pair of electrodes 32. The cover member 16 is built on the side of the second base member 15 opposite to the first base member 14. According to this configuration, the durability of the leads 37 is improved as compared to the case where the first base member 14 is not provided.

In the muscle electrostimulation device 10 according to the first embodiment, the main body 20 has a bifurcated shape in which a portion in the X directions is split in the Y direction by the gap 24g. The portion of the main body 20 adjacent to the gap 24g is provided with a thick portion 24h formed to have a larger thickness than elsewhere.
According to this configuration, the strength of the portion adjacent to the gap 24g is improved, troubles such as a crack are minimized, and the durability is improved.

In the muscle electrostimulation device 10 according to the first embodiment, the main body 20 is provided with the pair of belt joints 42 for attachment of the belt members 50. When one end 50b of a belt of the belt members 50 is joined to one of the pair of belt joints 42 and the main body 20 is wrapped around the portion fitted with the device, the other of the pair of belt joints 42 is located in a range in which the folded-back portion 50e of the belt of the belt members 50 can be guided through the other of the pair of belt joints 42. According to this configuration, the folded-back portion 50e of the belt of the belt members joined to one of the belt joints 42 is automatically located near the other of the pair of belt joints 42 so that the device can be easily fitted to the body.

In the muscle electrostimulation device 10 according to the first embodiment, the main body 20 is provided with the control unit 30 for supplying a voltage to the pair of electrodes 32. The pair of electrodes 32 are arranged in symmetry with respect to the control unit 30. According to this configuration, the main body 20 can be worn such that the control unit 30 is inwardly arranged on the body whether to train the muscle on the front of the portion fitted with the device or the muscle on the back of the portion. By inwardly arranging the control unit 30 on the body, the likelihood that the control unit 30 collides with, for example, a leg of a desk and is damaged is reduced. By inwardly arranging the control unit 30 on the body, the user can sense that the control unit 30 is located toward the center of the body at all times and is saved from the trouble of looking for the control unit 30.

### [Second embodiment]

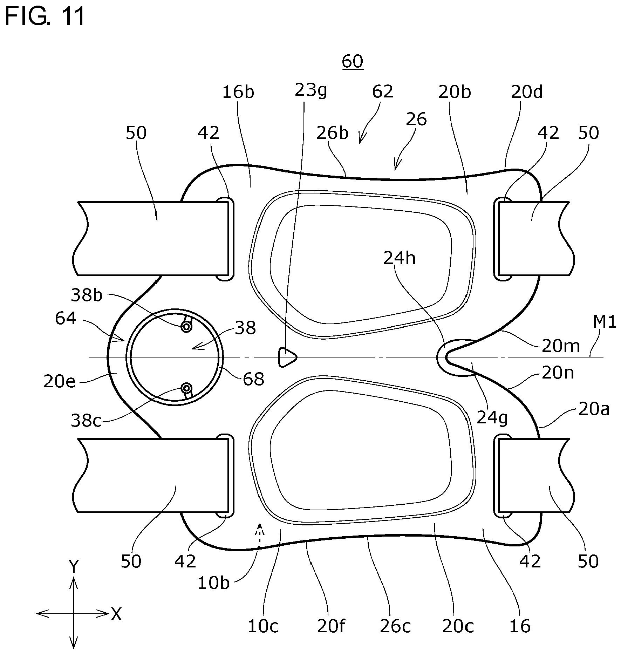

A description will be given of a muscle electrostimulation device 60 according to the second embodiment with reference to Figs. 11-13. In the drawings and description of the second embodiment, constituting elements and members identical or equivalent to those of the first embodiment shall be denoted by the same reference numerals. Duplicative explanations are omitted appropriately and features different from those of the first embodiment will be highlighted.

The control unit 30 of the muscle electrostimulation device 10 according to the first embodiment is described as being joined to the main body 20 in an integrated manner, but the invention is non-limiting as to the feature. For example, the main body may not be provided with a control unit and the control unit maybe configured as a separate device. The control unit may be configured to be detachably mounted by using an attachment mechanism provided in the main body.

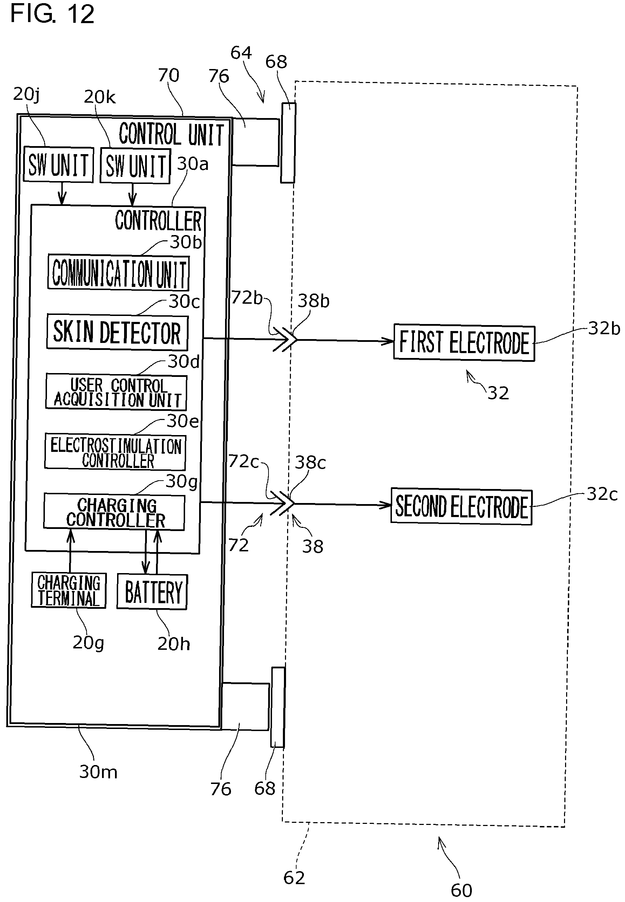

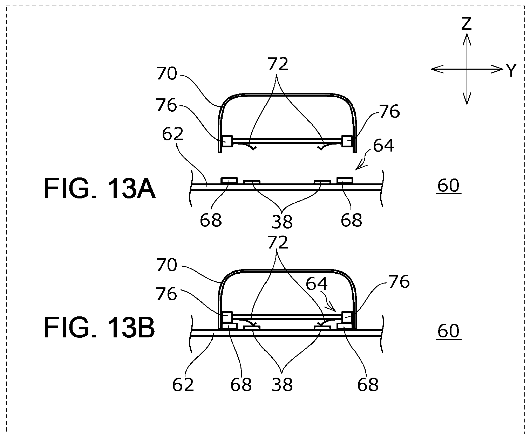

Fig. 11 is a front view of a main body 62 of the muscle electrostimulation device 60 according to the second embodiment. The muscle electrostimulation device 60 is smaller in size than the muscle electrostimulation device 10 according to the first embodiment and can be suitably used for the muscle in the upper limb such as the biceps muscle of arm and the triceps muscle of arm, and muscle of the lower limb of a slim body. Fig. 12 is a block diagram showing features of the muscle electrostimulation device 60. Figs. 13A and 13B are side views schematically showing the vicinity of an attachment mechanism 64 of the muscle electrostimulation device 60. Figs. 13A and 13B show an example of the control unit 70 that can be attached to the muscle electrostimulation device 60.

The muscle electrostimulation device 60 according to the second embodiment is not provided with a control unit in the main body 62 and is provided with the attachment mechanism 64 and power receiving terminals 38. The control unit 70 is configured as a device separate from the main body 62 and is attached to the main body 62 by the attachment mechanism 64. The control unit 70 is provided with power feeding terminals 72. When the control unit 70 is mounted to the main body 62, the power feeding terminals 72 come into contact with the power receiving terminals 38 and supply electric power for electrostimulation to the power receiving terminals 38.

The main body 62 of the second embodiment corresponds to the main body 20 of the first embodiment and is provided with all features and characteristics other than the control unit 30. The control unit 70 supplies a voltage to the pair of electrodes 32. The control unit 70 corresponds to the control unit 30 of the first embodiment and is provided with all features and characteristics of the control unit 30.

### (Contact force reduction portion)

If a joint is bent while the muscle electrostimulation device 60 is being fitted to the upper limb or the lower limb, the vicinity of the center of the outer edge of the main body in the Y direction may come into hard contact with the joint, giving the user an uncomfortable tactile sensation. In particular, given the short distance from the outer edge of the main body 62 to the elbow joint occurring when the device is fitted to the upper limb, an outer edge 20d may come into hard contact with the elbow joint when the elbow joint is bent. To address this, the muscle electrostimulation device 60 according to the second embodiment is provided with a contact force reduction portion 26 in an area of the outer edge of the main body 62 that comes into contact with the joint near the portion fitted with the device when the joint is bent so as to reduce a force of contact with the joint.

For example, the contact force reduction portion 26 may include, in an area in the outer edge facing the center of the joint, a receding portion that recedes away from the joint. In the main body 62 of this example, the contact force reduction portion 26 includes arc-shaped receding portions 26b, 26c shaped to recede in the shape of an arc. By providing the arc-shaped receding portions 26b, 26c, outer edges 20d, 20f present a curved shape as shown in Fig. 11. The arc-shaped receding portions 26b, 26c are provided on the respective outer edges 20d, 20f of the main body 62 in the Y direction. Accordingly, the contact force can be reduced even when the main body 62 is worn upside down.

### (Attachment mechanism)

The main body 62 is provided with the attachment mechanism 64 and the power receiving terminals 38. The attachment mechanism 64 is a mechanism for detachably attaching the control unit 70 to the main body 62. The attachment mechanism 64 may be configured such that the control unit 70 can be attached or detached using a tool. It is desired that the control unit 70 can be attached or detached manually without using a tool. An attachment mechanism based on any of various principles such as that of general-purpose fasteners may be employed to configure the attachment mechanism 64 in the main body 62. One example of the attachment mechanism 64 that can be employed is a mechanism using magnetic attraction, a mechanism including a hook and a recess engageable with the hook, a mechanism including an external thread and an internal thread, a mechanism including a hook-and-loop fastener, and a mechanism including a snap button.

In the attachment mechanism 64 of this example, the control unit 70 is provided with a magnet 76, and the main body 62 is provided with a magnetic member 68 that attracts the magnetic pole of the magnet 76 magnetically. The magnetic attraction between the magnetic pole of the magnet 76 and the magnetic member 68 maintains the control unit 70 in the main body 62. When a force beyond the attraction of the magnet 76 is exerted on the control unit 70, the control unit 70 is removed from the main body 62. Fig. 13A shows the muscle electrostimulation device 60 in which the control unit 70 is not attached. Fig. 13B shows the muscle electrostimulation device 60 in which the control unit 70 is attached.

### (Power receiving terminals)

The power receiving terminals 38 include two terminals 38b, 38c arranged at a predetermined interval in the main body 62. As in the first embodiment, the terminals 38b, 38c are connected to the first electrode 32b and the second electrode 32c of the pair of electrodes 32. The surface of the terminals 38b, 38c may be treated to prevent rust on the electrode surface. For example, the terminals may be plated with noble metal. The power receiving terminals 38 may be configured as a spring member in order to stabilize the pressure of contact with the power feeding terminals 72.

### (Power feeding terminals)

The power feeding terminals 72 include terminals 72b, 72c arranged at positions aligned with the terminals 38b, 38c of the power receiving terminals 38. For example, the terminals 72b, 72c may be configured as a spring member. The terminals 72b, 72c supply electric power for electrostimulation to the terminals 38b, 38c.

As described in the first embodiment, the main body 20 may be arranged in symmetry with respect to the control unit 70. In particular, the main body 62 may be formed in line symmetry with respect to the straight line M1. The pair of electrodes 32 may be arranged in line symmetry with respect to the control unit 70. In particular, the pair of electrodes 32 may be arranged in line symmetry with respect to the straight line M1.

### (Storage method)

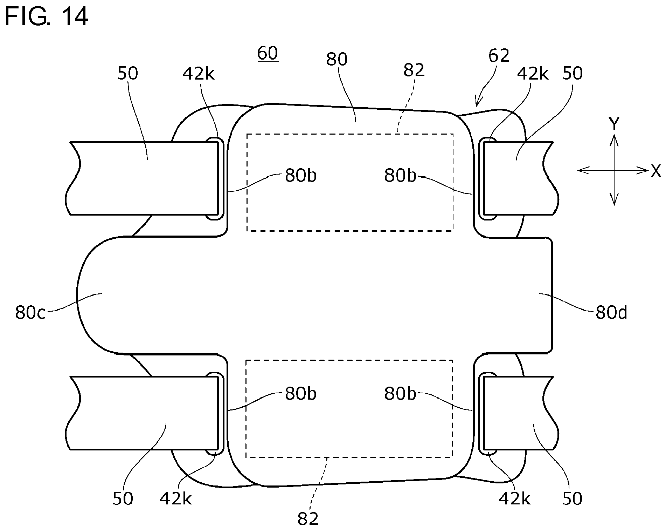

A description will now be given of an exemplary method of storing the muscle electrostimulation device 60 with reference to Fig. 14. Fig. 14 is a rear view showing a mount sheet 80 pasted to the muscle electrostimulation device 60. The muscle electrostimulation device 60 may be stored with the gel pad 82 pasted on the rear side after use. Therefore, the mount sheet 80 having certain rigidity may be pasted on the rear side of the gel pad 82.

The mount sheet 80 may be size larger than the main body 62 and may be shaped in a rectangle. It is desired that the device can be stored with the belt members 50 being attached. To address the requirement, the mount sheet 80 is provided with a cutout portion 80b in areas that the rectangular tube members 42k and the belt members 50 come into contact. By providing the cutout portion 80b, the mount sheet 80 can be intimately attached to the gel pad 82 at a distance from the rectangular tube members 42k and the belt members 50. The portions of the mount sheet 80 excluding the cutout portions 80b at the four corners constitute extensions 80c, 80d that extend on the respective sides in the X direction. The surface of the mount sheet 80 may be coated for smoothing.

The muscle electrostimulation device 60 may be pasted to the mount sheet 80 and stored in a storage bag. The storage bag may be formed from a cloth or a resin sheet. In this way, the device can be stored with the gel pad 82 being attached, and the likelihood of foreign materials or the bag being glued to the gel pad 82 is reduced.

The muscle electrostimulation device 60 according to the second embodiment configured as described above operates similarly to the muscle electrostimulation device 10 according to the first embodiment when the control unit 70 is attached.

The muscle electrostimulation device 60 according to the second embodiment provides the same advantage and benefit as those of the muscle electrostimulation device 10 according to the first embodiment. In addition, the muscle electrostimulation device 60 according to the second embodiment provides the following advantage and benefit.

In the muscle electrostimulation device 60 according to the second embodiment, the contact force reduction portion 26 for reducing a force of contact with the joint is provided in the area of the outer edge of the main body 62 that comes into contact with the joint near the portion fitted with the device when the joint is bent. According to this configuration, the force of contact with the bent joint can be reduced as compared with the case where the contact force reduction portion is not provided. This can mitigate uncomfortable tactile sensation at the portion fitted with the device caused by the contact force.

The main body 62 of the muscle electrostimulation device 60 according to the second embodiment is provided with the attachment mechanism 64 for detachably attaching the control unit 70 that supplies a voltage to the pair of electrodes 32. According to this configuration, one control unit may be switchably used in different types of main bodies. Further, if the main body goes out of order, only the main body may be replaced. This is more economical and saves more resources than when the control unit is not replaceable.

The invention is described above based on the embodiments. The embodiments are intended to be illustrative only and it will be understood to those skilled in the art that variations and modifications are possible within the claim scope of the present invention and that such variations and modifications are also within the claim scope of the present invention. Therefore, the description in this specification and the drawings shall be treated to serve illustrative purposes and shall not limit the scope of the invention.

A description will now be given of variations. In the drawings and description of the variations, constituting elements and members identical or equivalent to those of the embodiments shall be denoted by the same reference numerals. Duplicative explanations are omitted appropriately and features different from those of the embodiments will be highlighted.

### (First variation)

In the embodiments, the belt members 50 are described as being used to fit the muscle electrostimulation device to the body, but the embodiments are non-limiting as to the feature. The stickiness of a gel pad may be used to attach the muscle electrostimulation device to the body.

### (Second variation)

In the embodiments, the control unit 30 is described as being provided toward the end in the X direction, but the embodiments are non-limiting as to the location. The control unit 30 may be provided in an intermediate area of the main body 20 in the X direction. In particular, the center of the control unit 30 may be arranged at a position bisecting the main body 20 in the X direction.

### (Third variation)

In the embodiments, the longitudinal directions of the pair of electrodes 32 are described as being arranged substantially parallel to each other, but the embodiments are non-limiting as to the arrangement. The pair of electrodes may be arranged at an angle to each other. By way of example, the distance between the pair of electrodes may be configured to be progressively smaller from one end in the Y direction toward the other end. With this configuration, it is easy to arrange each of the pair of electrodes to conform to the three-dimensional shape of the muscle or the joint of the portion fitted with the device. For example, the pair of electrodes may be arranged in the shape of a letter V with its apical end missing, by approximating one end of one of the pair of electrodes and one end of the other electrode.

In this case, if the angle formed by the longitudinal directions of the pair of electrodes 43 is too large, one end of one of the pair of electrodes 43 approaches one end of the other electrode excessively, resulting in an uneven electric current distribution and a narrow range in which the nerve is stimulated. In this background, the angle formed by the longitudinal directions of the pair of electrodes may be configured to be 90° or smaller. It is confirmed that practical performance is obtained in this range.

### (Fourth variation)

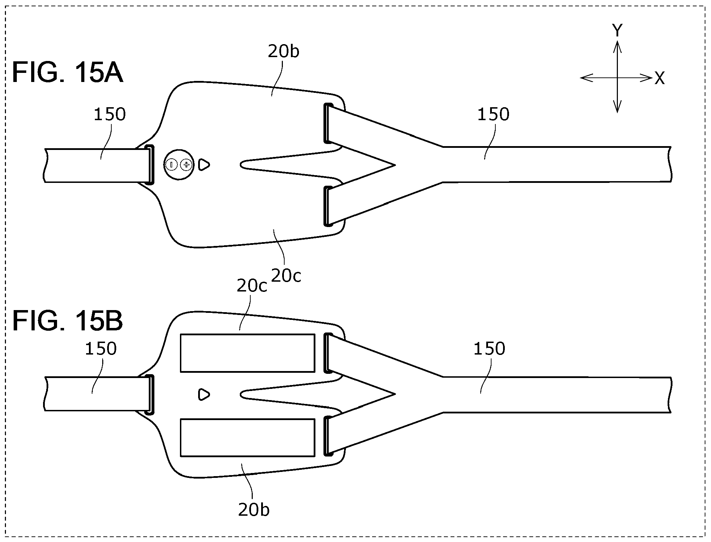

In the embodiments, an example is described in which the belt members 50 are respectively provided for the two band-shaped members 20b, 20c, and the two belt members 50 are independently wrapped around the portion fitted with the device, but the embodiments are non-limiting as to the feature. Alternatively, one belt member may be provided for the two band-shaped members 20b, 20c. Figs. 15A and 15B are schematic diagrams illustrating a belt member 150 according to the fourth variation. Fig. 15A is a schematic diagram showing a front view, and Fig. 15B is a schematic diagram showing a rear view. As shown in Figs. 15A and 15B, the belt member 150 according to the fourth variation has bifurcated, Y-shaped portions. The Y-shaped portions of the belt member 150 are respectively joined to the band-shaped members 20b, 20c and a single belt member 150 attaches the two band-shaped members 20b, 20c to the portion fitted with the device. By using the belt member 150 having Y-shaped portions in this way, the main body having a bifurcated structure highly adaptive to the body shape can be worn together.

### (Fifth variation)

In the embodiments, the remote controller 12 is described as being provided with a touch panel capable of display and touch operations, but the embodiments are non-limiting as to the feature. An input for user control may be entered in the remote controller 12 using a user control device other than a touch panel, based on an image displayed on a display device other than a touch panel. The display device is exemplified by a liquid display device and the user control device is exemplified by a key switch such as a keyboard.

The variations described above provide the same advantage and benefit as the embodiments.

Arbitrary combinations of the embodiments and combinations of an embodiment and a variation will also be useful as embodiments of the present invention. A new embodiment created by a combination will provide the combined advantages of the embodiment and the variation as combined.

## Claims

1. A muscle electrostimulation device (10) comprising:
a main body (20) having a contact surface (10b) fitted around a portion fitted with the device (10) of a lower limb (6) or an upper limb, wherein
the contact surface (10b) extends in an X direction encircling the portion fitted with the device (10) and in a Y direction perpendicular to the X direction,
the main body (20) is provided with a pair of electrodes (32) arranged at a distance in the Y direction, and
each of the pair of electrodes (32) is shaped in a band in which an extent in the X direction is larger than an extent in the Y direction and is arranged to cause an electric current to flow in the portion fitted with the device (10) along a direction of extension of a muscle fiber in the portion fitted with the device (10).

2. The muscle electrostimulation device (10) according to claim 1, wherein
the longitudinal directions of the pair of electrodes (32) are arranged substantially parallel to each other.

3. The muscle electrostimulation device (10) according to claim 1, wherein
a distance between the pair of electrodes (32) is configured to be progressively smaller from one end along the Y direction toward the other end.

4. The muscle electrostimulation device (10) according to one of claims 1 through 3, wherein
the main body (20) includes a first base member (14), a second base member (15), and a cover member (16),
the first base member (14) has an opening (14b) that exposes the pair of electrodes (32),
the second base member (15) is provided with the pair of electrodes (32), and
the cover member (16) is built on a side of the second base member (15) opposite to the first base member (14).

5. The muscle electrostimulation device (10) according to one of claims 1 through 4, wherein
the main body (20) is provided with a pair of joints for attachment of a belt member (50), and
when one end of the belt member (50) is joined to one of the pair of joints and the main body (20) is wrapped around the portion fitted with the device (10), the other of the pair of joints is located in a range in which the other end of the belt member (50) can be guided through the other of the pair of joints.

6. The muscle electrostimulation device (10) according to one of claims 1 through 5, wherein
the main body (20) is provided with a control unit (30) that supplies a voltage to the pair of electrodes (32), and
the pair of electrodes (32) are arranged in symmetry with respect to the control unit (30).
